# EUROPÄISCHE PATENTANMELDUNG

(11) **EP 2 851 662 A2**
(43) Veröffentlichungstag der Anmeldung: **25.03.2015**
(21) Anmeldenummer: 14180707.3
(22) Anmeldetag: 12.08.2014
(51) Int. Cl.: G01J 3/02, G01J 3/06, G01J 3/28

(54) **Vorrichtung und Verfahren zur Aufnahme eines Hyperspektralbildes**

(30) Priorität: 30.08.2013 DE 102013217379
(71) Anmelder: Spekled GmbH, 12833 Pepelow (DE)
(72) Erfinder: Kulcke, Axel, 18233 Pepelow (DE)
(74) Vertreter: UEXKÜLL & STOLBERG

(57) **Zusammenfassung**

Die Erfindung betrifft eine Vorrichtung zur Aufnahme eines Hyperspektralbildes eines Untersuchungsgebietes mit
- einer Lichtquelle (3) zur Bestrahlung des Untersuchungsgebietes,
- einem Eingangsobjektiv (2) zur Erzeugung eines Bildes des Untersuchungsgebietes in einer Bildebene,
- einem Spektrometer (11), das eine in der Bildebene angeordnete schlitzförmige Blende (7) zur Selektion eines schlitzförmigen Bereichs des Bildes, ein dispersive Element (9), das so aufgebaut und angeordnet ist, dass die dispersive Auffächerung des durch die Blende hindurch tretenden Lichts in einer von der Längsrichtung der Blende verschiedenen Richtung erfolgt, und einen Kamerasensor (10) zur Aufnahme des Beugungsbildes aufweist, und
- einer Datenverarbeitungseinrichtung zur Aufnahme der Kamerasensorsignale als eine Vielzahl von Spektren mit jeweils zugeordneter Ortskoordinate x entlang der Längsrichtung X der Blende,
- wobei die Vorrichtung dazu eingerichtet ist, in einer von der Längsrichtung X verschiedenen zweiten Richtung Y aufeinanderfolgende schlitzförmige Bereiche des Bildes des Untersuchungsgebietes mit zugeordneter Ortskoordinate y aufzunehmem,
- dadurch gekennzeichnet, dass das Spektrometer (11) in der zweiten Richtung Y verfahrbar relativ zum Eingangsobjektiv (2) gelagert ist, eine Antriebseinrichtung zum gesteuerten Verfahren und Einstellen der Position des Spektrometers (11) in der zweiten Richtung Y vorhanden ist und dass die Datenverarbeitungseinrichtung dazu eingerichtet ist, das Spektrometer (11) schrittweise zu verfahren, um so sukzessive aufeinanderfolgende schlitzförmige Bereiche mit Spektren mit zugeordneten Ortskoordinaten x, y aufzunehmen und zu dem Hyperspektralbild zusammenzusetzen.

## Beschreibung

Die vorliegende Anmeldung betrifft eine Vorrichtung zur Aufnahme eines Hyperspektralbildes eines Untersuchungsgebietes eines Körpers, mit einer Lichtquelle zur Bestrahlung des Untersuchungsgebietes mit Licht mit für die gewünschte spektroskopische Analyse geeigneter spektraler Verteilung, einem Eingangsobjektiv zur Erzeugung eines Bildes des Untersuchungsgebietes in einer Bildebene, einem Spektrometer, das eine in der Bildebene angeordnete schlitzförmige Blende zur Ausblendung eines schlitzförmigen Bereichs des Bildes des Untersuchungsgebietes, ein dispersive Element, das so aufgebaut und angeordnet ist, dass die dispersive Auffächerung des durch die Blende hindurch tretenden Lichts in einer von der Längsrichtung der Blende verschiedenen Richtung erfolgt, und einen Kamerasensor aufweist, der so aufgebaut und angeordnet ist, dass er ein Beugungsbild des dispersive Elements mit der Längsrichtung der Blende in einer ersten Richtung und der dispersiven spektralen Auffächerung des Lichtes in der von der ersten verschiedenen Richtung auf seiner Kamerasensorfläche aufnimmt, und einer mit dem Kamerasensor verbundenen Datenverarbeitungseinrichtung, die dazu eingerichtet ist, die von dem Kamerasensor empfangenen Signale als eine Vielzahl von Spektren mit jeweils zugeordneter Ortskoordinate x entlang der Längsrichtung X der Blende zu speichern, wobei die Vorrichtung dazu eingerichtet ist, in einer von der Längsrichtung X verschiedenen zweiten Richtung Y in der Bildebene aufeinanderfolgende schlitzförmige Bereiche des Bildes des Untersuchungsgebietes mit zugeordneter Ortskoordinate y in der zweiten Richtung aufzunehmem, und die Datenverarbeitungseinrichtung dazu eingerichtet ist, die Spektren jeweils zu speichern und die aufgezeichneten Spektren mit den zugeordneten Ortskoordinaten x, y zu einem Hyperspektralbild des Untersuchungsgebietes zusammenzufassen.

Das Hyperspektralbild kann man sich prinzipiell als dreidimensionalen Vektorraum vorstellen, in dem die Ortskoordinaten x, y zwei Dimensionen und die Wellenlängen λ die dritte Dimension aufspannen. Aufgrund der endlichen Anzahl von Sensorelementen auf dem Kamerasensor und der damit begrenzten Auflösung der Ortskoordinate x entlang Längsrichtung der Blende und der λ-Auflösung sowie der endlichen Ortsauflösung in der Ortskoordinate y durch die schrittweise Abtastung des Untersuchungsgebiets in Y-Richtung handelt es sich bei dem Hyperspektralbild in der Praxis aber nicht um einen Vektorraum, sondern um eine Menge von 3-Tupeln (xᵢ, yⱼ, λₖ).

Die Erfindung betrifft ferner ein entsprechendes Verfahren zur Aufnahme eines Hyperspektralbildes eines Untersuchungsgebietes eines Körpers.

Im wissenschaftlich-technischen Bereich der Spektroskopie haben sich in den letzten ca. 20 Jahren viele Anwendungen weiterentwickelt. Dabei gewann die ortsaufgelöste Spektroskopie zunehmend an Bedeutung. Die neuen Systeme und Verfahren sind unter den Bezeichnungen Spectral Imaging, Hyperspectral Imaging, Chemical Imaging oder hyperspektrale Kameras bekannt. Hyperspektrale Kameras zeichnen sich gegenüber spektralen Kameras dadurch aus, dass sie ein in viele Wellenlängenpunkte zerlegtes, quasikontinuierliches Spektrum aufzeichnen, während (multi-)spektrale Kameras nur wenige spektrale Stützstellen, die auch noch spektral separiert voneinander liegen können, erfassen.

Mit den hyperspektralen Verfahren werden den Nutzern wesentliche und neue Möglichkeiten zur Analyse oder Diagnose von Bilddaten an die Hand gegeben. Es wäre hierbei auch sehr wichtig, dass für einen Benutzer auch die mit dem eigenen Auge erkennbare Farbbildinformationen mit den für ihn unsichtbaren chemischen Informationen aus den Spektren kombiniert oder in Bildern dargestellt überlagert werden. In der Medizin ist dieser Schritt vergleichbar mit dem Schritt von der einfachen bildgebenden Diagnostik zur Computertomographie, in der dem Arzt eine zusätzliche Dimension der Datensätze zur Verfügung gestellt wird.

Hochauflösende Kamerasysteme, von allem Farbkamerasysteme mit kontinuierlicher schneller Bilderzeugung, also digitale Videosysteme, finden heute weit verbreitet Anwendung und sind Stand der Technik. Solche Systeme finden vielseitige Anwendung z.B. in der Automatisierungstechnik, Qualitätskontrolle, Medizintechnik und Mikroskopie. Hilfreich hierfür ist es, auch ortsaufgelöste spektrale Informationen zu erhalten, über die man chemische Informationen ortsaufgelöst in dem Untersuchungsgebiet erhalten kann. Dabei ist es häufig wichtig und sinnvoll, über den spektralen Empfindlichkeitsbereich des Auges von 400 nm bis 700 nm hinauszugehen. Im UV- und NIR-Bereich sind spektrale Informationen vorhanden, die direkt mit den chemischen Eigenschaften des untersuchten Körpers verknüpft sind. Quantenmechanisch sind es im UV-Bereich die Elektronenübergänge in den Elektronenhüllen von Atomen und Molekülen. Im IR-Spektralbereich sind es Vibrationsübergänge in den Molekülen. Sehr wichtig sind hier gerade der sehr nahe Infrarotbereich von 700 nm bis 1.100 nm und auf der anderen Spektralseite im UV-Bereich der Bereich zwischen 200 nm und 400 nm. Viele verfügbare Kamerasensoren (CMOS oder CCD) sind auch in diesen Spektralbereichen empfindlich.

Im Infrarotbereich wird überwiegend mit reiner Absorptionsspektroskopie gearbeitet, während im UV-Bereich heute vorwiegend Fluoreszenzspektroskopie eingesetzt wird. Dabei wird im Allgemeinen der zu untersuchende Körper mit UV-Licht bestrahlt, und es wird das längerwellige emittierte Fluoreszenzlicht vorwiegend im sichtbaren Bereich und im NIR-Spektralbereich analysiert.

Im sehr nahen Infrarotbereich (VNIR - 700 bis 1.200 nm) sind durch die relativ niedrigen Absorptionsquerschnitte organischer Moleküle und vor allem des Wassers die Eindringtiefen der Strahlung in den Körper höher als in längerwelligen Spektralbereichen. So haben chemische Ungleichgewichte an der Oberfläche in diesem Spektralbereich einen niedrigeren Einfluss. Daher wird in der Medizin und Biologie dieser Spektralbereich auch als "optisches Fenster" bezeichnet.

Für die Aufnahme von spektral aufgelösten Bildern sind unterschiedliche Vorrichtungen bereits bekannt.

Zum einen handelt es sich dabei um Systeme zur multispektralen Photometrie. Ein solches System für die Anwendung in der Medizintechnik ist z.B. in US 6,640,132 beschrieben. Das System umfasst ein Filterrad, das schrittweise gedreht wird, um sukzessive verschiedene Filter in den Strahlengang zu bringen und Bilder mit dem jeweils selektierten Wellenlängenbereich aufzunehmen. Solche Filterradsysteme sind aber auf wenige Stützstellen des Spektrums beschränkt. Ferner nehmen sie Bilder bei verschiedenen Wellenlängen sequenziell auf, was in der Praxis zu bestimmten Problemen führt, worauf später noch eingegangen wird.

Eine vom Prinzip her ähnliche Möglichkeit, aufeinanderfolgend Bilder in verschiedenen Wellenlängenbereichen aufzunehmen, bietet der Einsatz von durchstimmbaren optischen Filtern. Durchstimmbare Filter arbeiten z.B. auf Flüssigkristallbasis und werden auch als "Liquid Crystal Tunable Filters" (LCTFs) bezeichnet. Eine Ausführungsform mit einem solchen Filteraufbau ist zum Beispiel in US 6, 992, 809 B1 beschrieben. Ein Beispiel für eine Vorrichtung mit einem durchstimmbaren Filter für die Fluoreszenzspektroskopie ist in US 2012/061586 A1 beschrieben. Auch diese Systeme arbeiten mit einer sequenziellen spektralen Aufnahme, was die spektrale Bildaufnahme relativ langsam macht, und haben im Allgemeinen einen geringen Strahlungsdurchsatz.

Hyperspektrale Kameravorrichtungen auf Basis von bildgebenden Spektrometern werden im Allgemeinen mit einem abbildenden Transmissions- oder Reflexionsspektrometer aufgebaut, das jeweils für einen streifenförmigen Bereich des Bildes des Objekts die zugehörigen Spektren erzeugt. Zum Aufbau eines zweidimensionalen Bildes aus sukzessiven Aufnahmen aufeinanderfolgender streifenförmiger Bereiche des Bildes des Untersuchungsgebietes wird im Allgemeinen eine durchfahrbare Spiegeleinheit zwischen Objektiv und Objekt, meistens direkt in der Nähe des Objektivs, eingesetzt oder es wird ein mechanischer Vorschub des Objektes realisiert, bei dem das zu untersuchende Objekt an dem feststehenden Spektrometer schrittweise vorbeibewegt wird. Ein solches bildgebendes Spektrometer ist in US 2012/0105845 A1 beschrieben; dieser Stand der Technik bildet die Grundlage für die Oberbegriffe von Anspruch 1 und Anspruch 11. Das Spektrometer hat eine schlitzförmige Blende zur Ausblendung eines schlitzförmigen Bereiches des Bildes des Untersuchungsgebietes. Das durch die Blende fallende Licht eines schlitzförmigen Bereichs des Bildes des Untersuchungsgebiets fällt auf ein dispersives Element, das als Prisma oder Gitter ausgebildet sein kann, das so aufgebaut und angeordnet ist, dass die dispersive Auffächerung des durch die schlitzförmige Blende hindurchtretenden Lichts in einer von der Längsrichtung der Blende verschiedenen Richtung, z.B. senkrecht dazu, erfolgt. Ein Kamerasensor nimmt das Beugungsbild auf, wobei in dem Beugungsbild auf der Kamerasensorfläche die Längsrichtung der Blende in einer ersten Richtung und die dispersive spektrale Auffächerung des Lichtes in der von der ersten verschiedenen Richtung liegt. Die erste Richtung enthält nun Ortsinformation des Bildelements entlang der Längsrichtung der schlitzförmigen Blende, während in der zweiten Richtung die spektrale Auffächerung des Lichts des zugehörigen Bildelements verläuft. Die Richtungen der Ortsinformation und der Wellenlängeninformation können senkrecht zueinander stehen, es können aber auch schiefwinklige Koordinatensysteme verwendet werden, bei denen die Orts- und Wellenlängeninformationen später durch Umrechnung einander zugeordnet werden. Eine mit dem Kamerasensor verbundene Datenverarbeitungseinrichtung ist dazu eingerichtet, die von dem Kamerasensor empfangenen Signale als eine Vielzahl von Spektren mit jeweils zugeordneter Ortskoordinate x entlang der Längsrichtung X der Blende zu speichern. Die Vorrichtung ist dazu eingerichtet, in einer von der Längsrichtung X verschiedenen zweiten Richtung y in der Bildebene aufeinanderfolgende schlitzförmige Bereiche des Bildes des Untersuchungsgebietes mit zugeordneter Ortskoordinate y in der zweiten Richtung Y aufzunehmen, wobei die Datenverarbeitungseinrichtung dazu eingerichtet ist, die Spektren jeweils zu speichern und die aufgezeichneten Spektren mit den zugeordneten Ortskoordinaten x, y zu einem Hyperspektralbild des Untersuchungsgebietes zusammenzufassen. Die aufeinanderfolgende Aufnahme sukzessiver streifenförmiger Bereiche des Untersuchungsgebietes erfolgt dadurch, dass das zu untersuchende Objekt entlang der zweiten Richtung Y an dem Spektrometer vorbeibewegt wird, wobei nach jedem Vorschubschritt des Antriebs eine Aufnahme eines streifenförmigen Bereichs des Untersuchungsgebietes vorgenommen wird und die Vorschubschrittweite der Breite des streifenförmigen Bereiches entspricht.

Die vorliegende Erfindung ist auf eine hyperspektrale Kameravorrichtung und ein hyperspektrales Aufnahmeverfahren zur Anwendung in der Medizintechnik gerichtet. Ein typisches Anwendungsgebiet ist die Untersuchung der Sauerstoffsättigung in einem Untersuchungsgebiet eines Körperteils eines Patienten. Zur Untersuchung der Sauerstoffsättigung im Blut und Gewebe wird die NIR-Spektroskopie angewendet. Die Sauerstoffsättigung ist einer der wichtigsten Überwachungsparameter, da eine Minderversorgung der Zellen zu Schädigungen oder Absterben führen kann. Der Sauerstofftransport im Körper erfolgt über das Hämoglobin im Blut. Ein weiterer wichtiger Messparameter ist die Hämoglobinkonzentration im Blut, im Gewebe oder im jeweils spezifischen Organ. Alle diese Parameter werden heute üblicherweise noch mit invasiver Blutabnahme und einer spektrometerbasierten Blutgasanalyse erfasst. Kontinuierlich und nicht invasiv können diese Parameter über Pulsoximeter (arterielle Sauerstoffsättigung) oder Gewebeoximeter erfasst werden. Diese Oximeter benutzen Sensoren, die punktuell an einer Messstelle die Daten erfassen. Übliche Messstellen am menschlichen Körper sind Finger oder die Stirn.

Sehr häufig ist es wichtig, nicht nur einen Messpunkt zu untersuchen, sondern ein ortsaufgelöstes Oxygenierungsbild eines Körperteils oder eines Organs zu erfassen. Weiterhin sollte die Aufnahme kontinuierlich oder quasikontinuierlich durchführbar sein, damit direkte Veränderungen bei bestimmen Therapien oder operativen Eingriffen verfolgt werden können.

Ein ortsaufgelöstes Oxygenierungsbild mit zusätzlichen chemometrischen Auswertungen nach Hämoglobingehalt, Wassergehalt, Fettgehalt, aber auch die Erfassung von Knochen- und Gewebeeigenschaften bietet eine Hilfestellung bei einer Vielzahl von medizinischen Aufgabestellungen.

So konnte bereits in unterschiedlichen Veröffentlichungen nachgewiesen werden, das Tumorgewebe von normalem Gewebe in vivo mit Methoden der hyperspektralen Bilderfassung unterschieden werden kann (S. Panasyuk et al., "Medical Hyperspectral Imaging to Facilitate Residual Tumor Identification During Surgery, Cancer Biology & Therapy, 6:3, S. 439-446, März 2007, und H. Akbari et al., "Hyperspectral Imaging and Quantitative Analysis for Prostate Cancer Detection", J. Biomed Opt. 2012 Juli, 17(7)).

Aber auch Wundheilungsverläufe, arterielle Verschlusskrankheiten und Geschwüre bei diabetischen Füßen können von Ärzten unter zur Hilfenahme der hyperspektralen Bildinformationen umfassender beurteilt werden.

Im UV-Spektralbereich konnten fluoreszenzspektroskopische Untersuchungen an Bakterien und Keimen zeigen, dass auch in diesem Spektralbereich interessante Anwendungen für erfindungsgemäße Vorrichtungen und Verfahren vorhanden sind (M. Son et al., "Fluorence Spectroscopy for Rapid Detection and Classification of Bacterial Pathogens", Applied Spectroscopy 63, 11 (2009), S. 1251-1255, und W. Jon et al., "Microbial Biofilm Detection on Food Contact Surfaces by Macro-Scale Fluorescence Imaging", Journal of Food Engineering 99 (2010), S. 314-322).

Für medizintechnische Anwendungen stellt sich in der Praxis und im klinischen Alltag ein komplexes Anforderungsprofil an eine hyperspektrale Kameravorrichtung. Eine solche hyperspektrale Kameravorrichtung muss zunächst so kompakt sein, dass sie einfach über der zu untersuchenden Stelle des Patienten positioniert und dort fixiert gehalten werden kann. Als typische maximale Richtwerte können hier Abmessungen von 20 x 20 x 20 cm³ und ein Gewicht von ca. 5 kg angenommen werden, damit die Vorrichtung an üblichen Gerätschaften im klinischen Bereich einsetzbar ist. Diese Richtwerte beziehen sich auf die Gesamtvorrichtung inklusive Beleuchtung, Objektiv, Spektrometer, Datenverarbeitungseinrichtungen und Gehäuse. Weiterhin muss die Bildfolge in kurzen Abständen aufgenommen werden. Dies ist notwendig, um ein Bild an einer Position zum einen scharf einzustellen und um zum anderen zu vermeiden, dass sich das zu untersuchende Körperteil während der Aufnahme bewegt. Für die Scharfstellung ist eine kontinuierliche Sequenz mit mindestens 10 Hz notwendig. Hierzu wäre es vorteilhaft, neben der hyperspektralen Aufnahme auch noch ein optisches Farbkamerabild des Untersuchungsgebietes aufzunehmen.

Das in der oben genannten Druckschrift US 2012/0105845 A1 beschriebene bildgebende Spektrometer ist für die Medizintechnik nicht geeignet und kann das beschriebene Anforderungsprofil nicht erfüllen, unter anderem da es sich um ein feststehendes Spektrometer handelt, an dem das zu untersuchende Objekt zur Bildaufnahme schrittweise vorbeibewegt werden muss.

Es ist Aufgabe der vorliegenden Erfindung, eine Vorrichtung und ein Verfahren zur hyperspektralen Bildaufnahme eines Untersuchungsgebietes eines Körpers für medizintechnische Anwendungen so zu verbessern, dass damit hyperspektrale Bildaufnahmen in Untersuchungsgebieten eines ruhenden Körpers vorgenommen werden können. Insbesondere sollen die Vorrichtung und das Verfahren unter klinischen Alltagsbedingungen einsetzbar sein.

Zur Lösung dieser Aufgabe dienen die hyperspektrale Aufnahmevorrichtung mit den Merkmalen des Patentanspruchs 1 und das hyperspektrale Aufnahmeverfahren mit den Merkmalen des Patentanspruchs 11. Vorteilhafte Ausführungsformen der Erfindung sind in den Unteransprüchen angegeben.

Erfindungsgemäß ist vorgesehen, dass das Spektrometer in der Bildebene in der zweiten Richtung y verfahrbar relativ zum Eingangsobjektiv gelagert ist. Die Vorrichtung bleibt dabei während einer Aufnahme stationär oder feststehend zu dem Körperteil über dem Untersuchungsgebiet, während das Spektrometer sukzessive in der Bildebene über das Untersuchungsgebiet bewegt wird. Die zweite Richtung y der Bewegung des Spektrometers kann dabei zu der ersten Richtung x (Längsrichtung der schlitzförmigen Blende) senkrecht stehen; grundsätzlich sind für die zweite Richtung y aber auch nicht-senkrechte, von der ersten Richtung x verschiedene Richtungen möglich, wobei sich dann im Falle einer nicht-senkrechten Verfahrrichtung y für die Form des aufgenommene Bild des Untersuchungsgebiets ein Parallelogramm ergibt. Es ist eine Antriebseinrichtung zum gesteuerten Verfahren und Einstellen der Position des Spektrometers in der zweiten Richtung y vorhanden. Die Datenverarbeitungseinrichtung ist dazu eingerichtet, das Spektrometer mit der Blende in der Bildebene des Untersuchungsgebietes entlang der Bildebene schrittweise sukzessive zu verfahren, um so sukzessive aufeinanderfolgende schlitzförmige Bereiche des Bildes des Untersuchungsgebietes mit Spektren der Bildelemente mit jeweils zugeordneten Ortskoordinaten x, y aufzunehmen und zu dem Hyperspektralbild zusammenzusetzen.

Durch die erfindungsgemäßen Maßnahmen ist es einerseits möglich, dass die Vorrichtung insgesamt feststehend oder stationär über dem Untersuchungsgebiet gehalten werden kann, während andererseits das Untersuchungsgebiet durch sukzessive schrittweise Bewegung des Spektrometers in der Bildebene des Eingangsobjektives abgetastet werden kann, wobei der Verfahrweg des Spektrometers deutlich kleiner als die Längsausdehnung des Untersuchungsgebietes ist; dies liegt daran, dass die Ausdehnung des Bildes des Untersuchungsgebiets in der Bildebene des Eingangsobjektivs in der Regel deutlich kleiner als die Ausdehnung des Untersuchungsgebietes selbst ist, wie dies auch für übliche optische Kameras bekannt ist, bei denen das Bild eines makroskopischen Objekt deutlich kleiner als das Objekt selbst ist.

In einer vorteilhaften Ausführungsform ist im Strahlengang zwischen dem Eingangsobjektiv und dem Spektrometer ein Strahlteiler angeordnet, der die Strahlen aus dem Untersuchungsgebiet hinter dem Eingangsobjektiv aufteilt und die abgeteilten Teilstrahlen ablenkt und auf einen zweiten Kamerasensor richtet, der in einer zweiten Bildebene angeordnet ist, um parallel zu dem Hyperspektralbild des Untersuchungsgebietes ein optisches Bild des Untersuchungsgebietes aufzunehmen und auf einer Anzeigeeinrichtung zur Anzeige zu bringen. Die Anzeige des optischen Bildes kann parallel ?zu Anzeigen zu dem Hyperspektralbild erfolgen oder es können Überlagerungen zwischen optischen und Hyperspektralbildern angezeigt werden.

In einer vorteilhaften Ausführungsform ist die Datenverarbeitungseinrichtung dazu eingerichtet, die Spektren des Hyperspektralbildes auf vorgegebene Kriterien , insbesondere charakteristischer spektraler Merkmale bestimmter Substanzen, wie z.B. Sauerstoff oder Hämoglobin, hin zu untersuchen und jedem Spektrum eines Bildpunktes nach Maßgabe des Ergebnisses der Untersuchung eine Bewertungsgröße zuzuordnen (z.B. eine Maßzahl, die das Vorhandensein von Sauerstoff bewertet) und eine zweidimensionale Darstellung des Untersuchungsgebietes zu erzeugen, in der jedem Bildelement in Abhängigkeit von der zugehörigen Bewertungsgröße eine optische Kodierung zugeordnet ist (z.B. eine Farbkodierung, die das Vorhandensein von Sauerstoff in einer Farbskala von 0 bis 100% abbildet), und so ein optisch kodiertes Analysebild in einer zweidimensionalen Form zu erzeugen (z.B. die ortaufgelöste Sauerstoffkonzentration) und auf der Anzeigeeinrichtung zur Anzeige zu bringen.

In einer bevorzugten Ausführungsform ist die Datenverarbeitungseinrichtung weiter dazu eingerichtet, neben einer oder mehreren optisch kodierten Formen von aus dem Hyperspektralbild gewonnenen Analysebildern auf der Anzeigeeinrichtung eine Darstellung des von dem zweiten Kamerasensor aufgenommenen optischen Farbbildes des Untersuchungsgebietes zur Anzeige zu bringen.

In einer bevorzugten Ausführungsform ist das dispersive Element des Spektrometers ein Gitter. Insbesondere ist es bevorzugt, als Gitter ein Blaze-Gitter zu verwenden, das dazu optimiert ist, das dispergierte Licht im Wellenlängenbereich von 500 bis 1.000 nm in einen vorgegebenen Winkelbereich konzentriert abzugeben, und dass das Blaze-Gitter ein Reflexions- oder Transmissionsgitter mit asymmetrisch sägezahnförmig gestalteter Oberfläche oder ein holographisches Gitter ist. Insbesondere ist es bevorzugt, als Blaze-Gittter ein VPH-Gitter (Volume Phase Holographic Grating) zu verwenden. Der Grund für die vorzugsweise Verwendung von Blaze-Gittern liegt besteht in Folgendem. Klassische Gitter haben den Nachteil, dass das Licht einer bestimmten Wellenlänge hauptsächlich in die 0-Ordnung geht und der Rest über höhere Ordnungen verteilt wird, was einem Verlust an Helligkeit und damit Empfindlichkeit in jeder einzelnen Ordnung mit sich bringt. Dieser Nachteil wird durch die sogenannten Blaze-Gitter überwunden. Blaze-Gitter sind dazu optimiert, das Licht nur in eine bestimmte Richtung und somit bei gegebenem Wellenlängenbereich hauptsächlich in eine bestimmte Ordnung zu beugen. Dadurch kann ein Kamerasensor in dem optimierten Winkelbereich positioniert werden und braucht dort nur einen relativ kleinen Raumwinkelbereich zu überdecken. Zu den ersten Blaze-Gittern gehörten Reflexionsgitter mit asymmetrisch sägezahnförmigen Oberflächengestaltungen, wobei die Sägezahnflanken als einzelne Spiegel jeweils so ausgerichtet sind, dass das Licht in Richtung der gewünschten Beugungsordnung reflektiert wird. Danach wurden auch holographische Gitter entwickelt, die den gleichen Effekt wie die zuerst beschriebenen Blaze-Gitter haben. Weitere Typen von Blaze-Gittern sind die später entwickelten sogenannten VPH-Gitter (Volume Phase Holographic Gratings). VPH-Gitter sind Transmissionsgitter, bei denen ein transparentes Material zwischen zwei Glas- oder Kunststoffscheiben eingeschlossen ist. In dem transparenten Material ist ein gewünschtes Muster eines variierenden Brechungsindex erzeugt, zum Beispiel durch holographische Belichtung und dadurch erfolgte Strukturänderungen des Materials. Ein Beispiel für ein solches Blaze-Gitter ist in dem Artikel "Volume-Phase Holographic Gratings and the Efficienty of Free Simple Volume-Phase Holographic Gratings", Samuel C. Barden et al., publications of the estronomical society of the passific, vol. 112, Seiten 809 - 820, Juni 2000, beschrieben. Mit solchen Blaze-Gittern lässt sich ein hoher Prozentsatz des gebeugten Lichts über den interessierenden Wellenlängenbereich in einem bestimmten Winkelbereich konzentrieren. Damit kann mit einem relativ kleinen Kamerasensor ein großer Anteil des Beugungsspektrums erfasst werden. Mit typischen Blaze-Gittern lassen sich ohne weiteres hohe Anteile von über 60% der Beugungsintensität in einem kleinen vorgegebenen Winkelbereich konzentrieren.

In einer bevorzugten Ausführungsform ist die Vorrichtung dazu eingerichtet, dass die Datenverarbeitungseinrichtung Zugriff auf vorab gemessene und gespeicherte Spektren von reinen, im Körper vorhandenen chemischen Substanzen (z.B. Sauerstoff, Hämoglobin, Fett etc.) hat und dazu eingerichtet ist, nach einem Verfahren der multivariaten Statistik die gemessenen Spektren eines Hyperspektralbildes aus den Anteilen der gespeicherten Spektren der chemischen Substanzen zusammenzusetzen und daraus Informationen über chemische Eigenschaften von Bildelementen zu bestimmen. Es sind viele multivariate statistische Analyseverfahren bekannt, um spektralen Messdaten zu analysieren, zum Beispiel Korrelation, Regression, Varianzanalyse, Diskriminanz-Analyse und Hauptkomponenten- und Faktoranalyse. Im vorliegenden Fall ist es bevorzugt, die Spektren von oxygeniertem Hämoglobin, deoxygeniertem Hämoglobin, Fett, Wasser und Proteinen gespeichert bereitzuhalten und die erfassten Spektren unter Verwendung dieser bekannten Spektren einer Hauptkomponentenanalyse zu unterziehen, um z.B. den relativen Anteil von Fett zu bestimmen.

Vorzugsweise ist die Datenverarbeitungseinrichtung dazu eingerichtet, mit den zweiten Ableitungen der aufgenommenen Spektren zu arbeiten. In den zweiten Ableitungen der Spektren treten die charakteristischen Merkmale der zu untersuchenden chemischen Substanzen signifikanter hervor, wohingegen gleichmäßige Hintergrund- oder Störeffekte in den zweiten Ableitungen weitgehend wegfallen.

Es ist ohne weiteres möglich, optische Farbbilder des Untersuchungsgebietes mit hoher Frequenz mit dem zweiten Kamerasensor aufzunehmen. Es kann dann auch vorgesehen sein, Bildvergleiche innerhalb einer Sequenz der aufgenommenen optischen Bilder vorzunehmen. Werden innerhalb der Sequenz Verschiebungen zwischen Untersuchungsgebiet und der Aufnahmevorrichtung entdeckt, also eine Bildverschiebung, so kann die Ausgabe eines parallel aufgenommenen Hyperspektralbildes blockiert werden. Somit lässt sich vermeiden, dass in den sequenziell aufgenommenen Bildern, aus denen das Hyperspektralbild zusammengesetzt wird, Bildverzerrungen auftreten.

Ferner wird durch die vorliegende Erfindung ein Verfahren zur Aufnahme eines Hyperspektralbildes eines Untersuchungsgebietes eines Körpers zur Verfügung gestellt, bei dem das Untersuchungsgebiet mit einer Lichtquelle mit Licht mit für die gewünschte spektroskopische Analyse geeigneter spektraler Verteilung bestrahlt wird, mit einem Eingangsobjektiv ein Bild des Untersuchungsgebiets in einer Bildebene erzeugt wird, mit einem Spektrometer, das eine in der Bildebene angeordnete schlitzförmige Blende zur Ausblendung eines schlitzförmigen Bereichs des Bildes des Untersuchungsgebietes, ein dispersives Element, vorzugsweise ein Gitter, das so aufgebaut und angeordnet ist, dass die dispersive Auffächerung des durch die Blende hindurch tretenden Lichts durch das Gitter in einer von der Längsrichtung der Blende verschiedenen Richtung erfolgt, und einen Kamerasensor aufweist, der so aufgebaut und angeordnet ist, dass er ein Beugungsbild des dispersiven Elements mit der Längsrichtung der Blende in einer ersten Richtung und der dispersiven spektralen Auffächerung des Lichtes in einer von der ersten verschiedenen Richtung auf seiner Kamerasensorfläche aufnimmt, eine Vielzahl von Spektren von Bildelementen entlang der Längsrichtung der Blende mit jeweils zugeordneter Ortskoordinate x entlang der Längsrichtung X der Blende aufgenommen wird, die aufgenommenen Spektren in einer mit dem Spektrometer verbundenen Datenverarbeitungseinrichtung mit jeweils zugeordneter Ortskoordinate x entlang der Längsrichtung X der Blende gespeichert werden, in einer von der Längsrichtung X verschiedenen zweiten Richtung Y in der Bildebene aufeinanderfolgende schlitzförmige Bereiche des Bildes des Untersuchungsgebietes mit zugeordneter Ortskoordinate y in der zweiten Richtung Y mit dem Spektrometer untersucht und die zugehörigen Spektren mit jeweils zugeordneten Ortkoordinaten x, y in der Datenverarbeitungseinrichtung gespeichert werden, die aufgezeichneten Spektren mit den zugeordneten Ortskoordinaten x, y zu einem Hyperspektralbild des Untersuchungsgebietes zusammengefasst werden.

Bei Anwendungen in der Medizintechnik sind in der Regel Raumbeleuchtungen für den Patienten und das medizinische Personal vorhanden. Diese Hintergrundbeleuchtung ist üblicherweise Tageslicht, Licht von Leuchtstofflampen, Halogenlampen oder anderen z.B. auf Weißlicht-LED basierenden Beleuchtungen. Somit ist immer eine Hintergrundbeleuchtung auf den Spektraldaten des Untersuchungsgebietes vorhanden. Diese Hintergrundbeleuchtung muss in zweierlei Hinsicht genau berücksichtigt werden. Zum einen kann sie während der Datenaufnahme variieren und zum anderen ist sie üblicherweise in ihrem Spektrum von der Lichtquelle, die zur Aufnahme der Spektren eingesetzt wird, verschieden. Somit ist es wichtig, dass zumindest die einzelnen Spektren einer Zeile des hyperspektralen Bildes in einer Belichtungszeit erfasst werden, in der die Hintergrundbeleuchtung sich nicht verändert. Wenn davon auszugehen ist, dass sich während der sequenziellen Aufnahme die Beleuchtungsbedingungen nicht ändern, kann eine Kalibration mithilfe von im Bild untergebrachten Bildmarkern oder auch durch eine Dunkelbildaufnahme vor und nach der Sequenzaufnahme zur Aufnahme des hyperspektralen Bildes durchgeführt werden. Weiterhin können bei Verwendung der zweiten Ableitung der Spektren zur chemometrischen Analyse konstante und lineare spektrale Anteile von einer Hintergrundbeleuchtung weitgehend eliminiert werden.

Als Kamerasensoren können zum Beispiel hoch integrierte Megapixel-CMOS-Bildsensoren verwendet werden. Typischerweise sind solche Sensoren in einer Farbsensor- und einer Monochromausführung verfügbar, die sich meist nur durch eine zusätzliche Farbfilterschicht über dem Sensorarray unterscheiden. Die Arraystruktur und die gesamte elektronische Beschaltung sind in beiden Ausführungsformen äquivalent. Da die geometrische Anordnung die gleiche ist, lassen sich so auch die Daten aus der Farbbildebene und der Hyperspektralbildebene sehr gut überlagern. Dies trifft vor allem zu, wenn die Abbildungsoptik der bildgebenden Spektrometereinheit eine 1:1 Abbildung liefert, d.h. wenn das optische Bild, das von dem zweiten Kamerasensor aufgenommen wird, und das Hyperspektralbild, das von dem ersten Kamerasensor aufgenommen wird, die gleiche Größe haben.

Weiterhin bieten diese hoch integrierten Sensoren viele Möglichkeiten der Parametrierung. So kann eine Teilbildregion (ROI = Region of Interest) definiert werden und damit muss aus dem Array zum Beispiel nur für diesen Bereich der gewünschte Spektralbereich ausgelesen werden. Weiterhin kann zur Reduzierung der Datenmenge eine Zusammenfassen von Bildelementen (Binning) und ein Überspringen von Bildelementen (Skipping) aktiviert werden. So kann die Auflösung des hyperspektralen Bildes in Orts- und Spektralrichtung einfach reduziert werden, was zu einer schnelleren Aufnahme der Hyperspektralbilder führt und damit die Erzeugungsfrequenz der sehr großen dreidimensionalen Datenarrays des Hyperspektralbildes stark erhöht. So kann z.B. die Auflösung des Hyperspektralbildes in Richtung jeder Achse um den Faktor 4 reduziert werden, was die Datenmenge um den Faktor 64 reduziert und somit eine starke Erhöhung der Frequenz ermöglicht. Zur Überlagerung lassen sich bei reduzierter Bildauflösung aber die Zwischenbereiche zum hoch aufgelösten Farbbild wiederum interpolieren.

Da die Sensoren häufig die analoge Verstärkung und auch die A/D Wandlung der Signale mit integrierten Schaltungen durchführen, lassen sich über einfache Parametrierungen der Verstärkung und der Belichtungszeit auch die Helligkeiten der Farbbilder und der Hyperspektralbilder gut anpassen.

Das Beugungsbild der Blende liegt z.B. so auf dem Kamerasensor, dass die Längsausdehnung der Blende der X-Richtung entspricht und die dispersive Auffächerung senkrecht dazu erfolgt. Dann entspricht jede Spalte des Kamerasensors einem Bildelement (oder einem Blendenlängenelement) des durch die Blende hindurch tretenden Lichts, dessen Spektrum in Y-Richtung entlang einer Spalte des Kamerasensors verläuft. Jede Spalte bildet dann ein unabhängig gemessenes Spektrum. Jedem Spektrum ist ein Ort entlang der Längsausdehnung der Blende zugeordnet. Auf diese Weise wird ein durch die Blende definierter schlitzförmiger Bereich des Bildes Untersuchungsgebietes spektral aufgelöst aufgenommen. Durch schrittweises sukzessives Bewegen des Spektrometers mit der Blende durch die Bildebene des Bildes des Untersuchungsgebietes kann dieses sukzessive abgetastet und ein hyperspektrales zweidimensionales Bild des Untersuchungsgebietes aufgebaut werden.

Die dispersive Auffächerung des durch die Blende hindurchtretenden Lichts durch das Gitter (oder ein anderes dispersives Element) muss nicht senkrecht zur Längsrichtung der Blende erfolgen. Es kann aber auch jeder andere von 0 und 180° verschiedene Winkel zwischen der Längsrichtung und der Richtung der spektralen Auffächerung liegen; in diesem Fall steht die λ-Achse (Richtung der spektralen Auffächerung) unter eben diesem Winkel zur Längsrichtung der Blende auf dem Kamerasensor. Jedem Punkt auf dem Kamerasensor kann dann eindeutig ein Wertepaar in dem (schrägwinkligen) Koordinatensystem aus λ-Achse und dem Ort in Längsrichtung der Blende zugeordnet werden.

Unter dem Begriff Blende sollen hier alle optischen Mittel verstanden werden, die das Bild bis auf einen langgestreckten, streifenförmigen Bereich von austretendem Licht ausblenden, wobei der streifenförmige Bereich nicht notwendig in Längsrichtung durchgängig sein muss, sondern auch aus einer reihenförmigen Folge von einzelnen separaten Bildelementen zusammengesetzt sein kann. Auch muss der langgestreckte streifenförmige Bereich nicht unbedingt linear sein.

In der Regel werden die Spektren bei der Analyse jeweils in Form ihrer weißnormierten Rohabsorptionsspektren und zusätzlich ihrer zweiten Ableitungen verwendet. Für eine Fluoreszenzspektroskopie wird die Aufnahme in relativen Rohintensitäten durchgeführt. Die Bildung der zweiten Ableitung der Spektren machen die Aufnahmen unabhängig von gerätespezifischen Einflüssen wie z.B. Beleuchtungsschwankungen oder aber auch von nicht spezifischen breitbandigen Absorptionsunterschieden, die im Körpergewebe von unterschiedlichen Personen vorliegen können. Weiterhin wird der Einfluss von spiegelnden Oberflächenreflexionen minimiert, da diese in den zweiten Ableitungen verschwinden.

Die Lichtquelle kann konventionell mit einer thermischen Lichtquelle (z.B. Halogenlampe) ausgestattet sein. Bevorzugt wird aber eine Lichtquelle, die auf LEDs basiert. Damit der jeweils festgelegte Spektralbereich kontinuierlich und homogen ausgeleuchtet werden kann, wird der Einsatz einer LED-Sorte mit kürzerer Wellenlänge bevorzugt, die mit einem breitbandigen Fluoreszenzfarbstoff überzogen ist, der in dem gewünschten Spektralbereich die benötigte kontinuierliche Strahlung erzeugt. Wichtig ist, dass die Strahlung möglichst intensive, breitbandig, temperaturstabil und in möglichst schnellen Pulsen erzeugbar ist, so dass nur während der jeweiligen Belichtungszeit des Sensors Licht auf das Objekt eingestrahlt werden muss. Da Halogenlampen, abgesehen von der breitbandigen Beleuchtung, diese Kriterien nur bedingt erfüllen können, sind die LED-Varianten für die vorliegende Erfindung eindeutig zu bevorzugen. Weiterhin muss der Strahlungseintrag auf den Körper im Untersuchungsgebiet gerade im medizinischen Bereich möglicht gering gehalten werden. So sollte das Emissionsspektrum der Lichtquelle möglichst auf das Aufnahmespektrum der Hyperspektralkamera angepasst sein, d.h. das Emissionsspektrum (ein thermischer Strahler strahlt viel mehr im langwelligen Bereich) soll zu der den Effizienzkurven von Gitter und Kamerasensor passen; schließlich kann auch noch die Absorptionskurve des Körperteils z.B. Haut berücksichtigt werden, die im Bereich 500 - 600 nm viel und im Bereich 600 - 950 nm wenig absorbiert.

Die Lichtquelle ist vorzugsweise mit optischen Elementen versehen, die das Licht der Lichtquelle geometrisch auf das Untersuchungsgebiet bündeln. Weiterhin können andere Elemente wie Polarisatoren oder Filter für eine Optimierung der Beleuchtung des Untersuchungsgebietes eingesetzt werden.

Die Erfindung wird im Folgenden anhand eines Ausführungsbeispiels in den Zeichnungen beschrieben, in denen:
Fig. 1 eine schematische Prinzipdarstellung zum Aufbau der Vorrichtung zur Aufnahme von Hyperspektralbildern zeigt,
Fig. 2 eine schematische Teildarstellung der Vorrichtung aus Fig. 1 zeigt,
Fig. 3 ein Blockschaltbild zur Erläuterung der Funktionsweise der Vorrichtung zeigt,
Fig. 4 NIR-Absorptionsspektren von für die spektroskopische Beurteilung wichtigen Substanzen des menschlichen Körpers, nämlich oxygeniertes Hämoglobin (HbO₂), nicht oxygeniertes Hämoglobin (HHb), Wasser (H₂O), Melanin, Fett und die Gewebestreuung zeigt und
Fig. 5 das Spektrum einer typischen Breitbandfluoreszenz-LED zeigt, die als Lichtquelle in der Vorrichtung zur Aufnahme medizinischer Hyperspektralbilder eingesetzt werden kann.

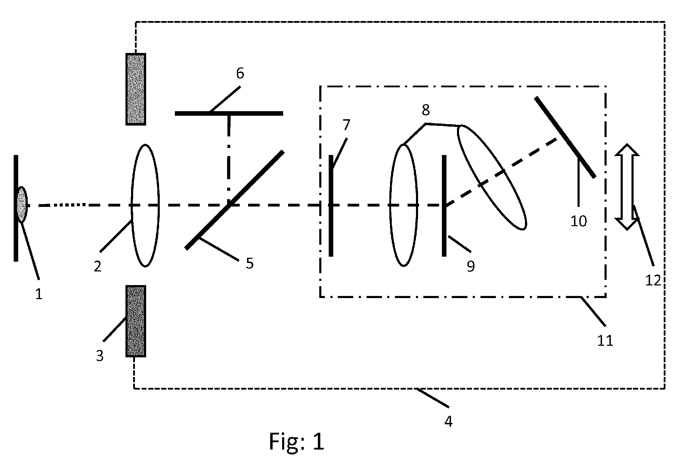

Fig. 1 zeigt als schematische Prinzipdarstellung den Aufbau einer erfindungsgemäßen Vorrichtung. Das zu untersuchende Körperteil oder Objekt ist mit 1 bezeichnet. Dieses kann zum Beispiel ein menschliches Körperteil oder Organ, grundsätzlich aber auch jedes andere Objekt oder jede andere Materialprobe sein. Das Bild des Untersuchungsgebietes wird mit einem Eingangsobjektiv 2 in eine Bildebene abgebildet.

Die Lichtquellen 3, die zu beiden Seiten des Eingangsobjektivs 2 angeordnet sind, erzeugen Licht einschließlich des Spektralbereichs für eine Farbkamera und für die Absorptionsspektroskopie. Im speziellen Fall der Fluoreszenzspektroskopie müssen die Lichtquellen so abgestimmt sein, dass sie die Fluoreszenz in der Probe anregen können. Die Lichtquellen 3 sind vorzugsweise in ein lichtdichtes Gehäuse 4 der gesamten Vorrichtung integriert.

Das Licht von dem Körperteil oder Objekt 1 trifft nach Passieren des Eingangsobjektivs 2 auf einen Strahlteiler 5. Dieser Strahlteiler 5 kann z.B. eine teilbeschichtete Trennscheibe oder ein Prisma sein. Wichtig ist hierbei, dass sichtbares Licht zur Erzeugung des Farbbildes auf dem zweiten Kamerasensor 6 aus dem Strahlengang ausgekoppelt wird. Dabei ist es sinnvoll zu berücksichtigen, dass vorteilhaft nur ein geringer Anteil der Strahlung durch den Strahlteiler 5 ausgekoppelt wird, da das Spektrometer das zur Verfügung stehende Licht spektral aufspaltet und daher im Strahlengang zur bildgebenden Spektrometereinheit ein wesentlich höherer Strahlungsanteil benötigt wird.

Der zweite, nicht ausgekoppelte oder direkte Strahlengang trifft in dem Spektrometer 11 zunächst auf eine spaltförmige Blende 7, die einen langgestreckten, streifenförmigen Bereich des Lichtes passieren lässt. Diese spaltförmige Blende 7 ist genau wie der zweite Kamerasensor 6 in der Bildebene des Eingangsobjektivs 2 angeordnet. Die spaltförmige Blende ist so angeordnet, dass ihre Längsrichtung in der Darstellung von Fig. 1 senkrecht zur Figurenebene steht. Mit dieser spalte- oder schlitzförmigen Blende 7 wird ein schlitzförmiger Bereich aus dem durch das Eingangsobjektiv 2 erzeugten Bild des Untersuchungsgebietes selektiert. Der von der Blende 7 selektierte streifenförmige Bereich wird über ein abbildendes optisches Element (z.B. Objektiv oder Achromat) 8 auf ein Gitter 9 geworfen. Dieses Gitter 9 ist so aufgebaut und angeordnet, dass die dispersive, wellenlängenabhängige Auffächerung des gebeugten Lichts in einer von der Längsausdehnung der Blende 7 verschiedenen, vorzugsweise dazu senkrechten, Richtung erfolgt. In der Darstellung von Fig. 1 bedeutet dies, dass die Längsrichtung der Blende senkrecht zu Figurenebene steht, während die dispersive Auffächerung des Lichts in der Figurenebene erfolgt. Über ein weiteres abbildendes optisches Element 8 wird das Beugungsbild der Blende dann auf einen Kamerasensor 10 geworfen. Auf der Kamerasensoroberfläche verläuft dann das Beugungsbild der Blende 7 mit der Längsausdehnung der Blende in einer ersten Richtung (in dem dargestellte Beispiel senkrecht zur Figurenebene) und mit der dispersiven Auffächerung des Beugungsbildes in einer zweiten, in diesem Beispiel zur ersten senkrechten Richtung (in der Figurenebene). Auf der Kamerasensoroberfläche entspricht dann jede Kamerasensorzeile einem Bildelement entlang der Längsrichtung der spaltförmigen Blende, und entlang der Kamerasensorzeile liegt die spektrale Auffächerung dieses Bildelements vor. Durch die aufeinanderfolgenden Kamerasensorzeilen wird eine aufeinanderfolgende Abfolge von Spektren erfasst, die einer Abfolge von Bildelementen entlang der Längsausdehnung der spaltförmigen Blende 7 und somit der Bildelemente des Bildes des Eingangsobjektivs auf einem Streifen entsprechen. Durch Verfahren des Spektrometers 11 in eine von der Längsrichtung verschiedene zweite Richtung y kann die spaltförmige Blende in der Bildebene des Eingangsobjektivs 2 sequenziell das Untersuchungsgebiet abtasten und somit die Daten für das hyperspektrale Bild erfassen. Da das Bild des Eingangsobjektives sehr klein und der Kantenlänge des Kamerasensors 6 entspricht, muss das bildgebende Spektrometer 11 insgesamt nur über sehr kleine Weglänge verfahren werden, was durch den Doppelpfeil 12 angedeutet ist. Da somit in jedem Schritt das Spektrometer 11 nur um eine sehr kleine Weglänge verfahren werden muss, sind damit nur geringe mechanische Beschleunigungen verbunden. Das gesteuerte Verfahren des Spektrometers 11 wird durch eine Antriebseinrichtung mit üblichen miniaturisierten Elektromotoren und Steuerungen für diese ausgeführt, die wiederum von einer Datenverarbeitungseinrichtung gesteuert werden.

Die spaltförmige Blende selektiert zum Beispiel einen 15 - 30 µm breiten Streifen aus dem Bild des Untersuchungsgebietes. Das Spektrometer kann zum Beispiel zwei Objektive oder Achromatenanordnungen 8 aufweisen. Das erste Objektiv 8 in dem Spektrometer bildet die spaltförmige Blende 7 ins Unendliche ab. Hinter dem Eingangsobjektiv 2 liegt das Gitter 9, das eine dispersive Aufspaltung des Lichts in Richtung zum Beispiel senkrecht zur Längsausdehnung der spaltförmigen Blende bewirkt. Das zweite Objektiv 8 des Spektrometers macht wieder die Abbildung vom Unendlichen auf das reale Bild, welches nun aber durch das dazwischen liegende Gitter spektral aufgefächert ist und durch den Kamerasensor 10 aufgenommen wird.

Bei den Kamerasensoren 6 und 10 handelt es sich - vorzugsweise um hochauflösende, zweidimensionale CMOS-Digitalkamerasensoren. Dabei ist der zweite Kamerasensor 6 als Farbkamerasensor mit einem Farbfilterarray versehen, während der Kamerasensor 10 ein monochromer Sensor ist. Dieser ermöglicht es, mit einer einzigen Bildaufnahme gleichzeitig typisch 1.000 Spektren (ein Spektrum pro Zeile) mit einer Datentiefe von typisch 12 Bit aufzunehmen. Jedes der Spektren entspricht dann dem Spektrum eines Bildelementes der Blende, d.h. man kann sich die schlitzförmige Blende 7 in 1.000 Bildelemente entlang ihrer Länge unterteilt denken, wobei das Licht jedes dieser Bildelemente durch das Gitter dann auf eine Zeile des Kamerasensors abgebildet wird. Der Kamerasensor kann die Bildaufnahme bei voller Sensorausnutzung etwa 50-mal pro Sekunde wiederholen. Somit würde die Erzeugung eines maximal aufgelösten Spektralbildes circa 20 Sek. Dauern. Zur Analyse werden in der Regel nur kleine Spektralbereiche herangezogen. Dies kann dadurch ausgenutzt werden, dass dann die bei den Kamerasensoren mögliche Teilbildaufnahme eingesetzt wird, um die Aufnahme zu beschleunigen. Bei CMOS-Sensoren können Teilbilder (regions of interest - ROI) eingestellt werden, die es ermöglichen, nur den eingestellten interessierenden Bildbereich des Sensors bei gleichzeitiger Beibehaltung der Grunddatenrate auszulesen. Weiterhin kann durch die Methode des Binnens oder Skippens die Auflösung reduziert werden, wodurch hyperspektrale Bilder in Aufnahmezeiten von einer Sekunde oder weniger erfasst werden können.

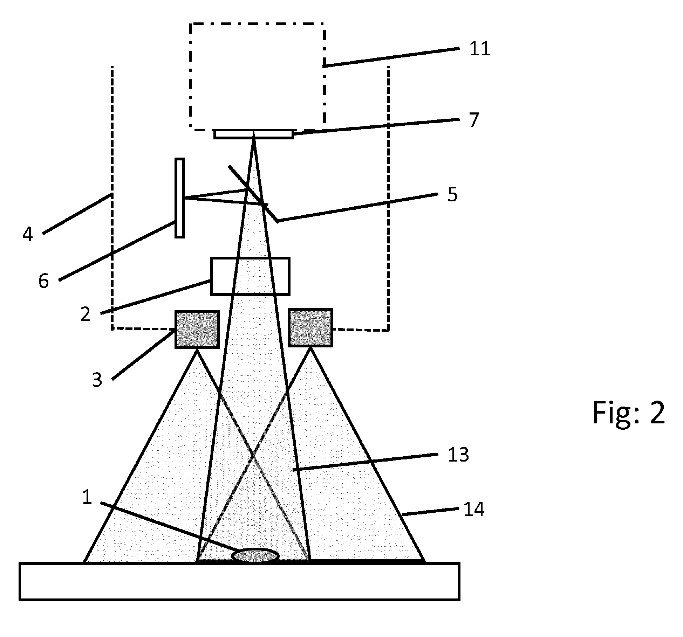

Fig. 2 zeigt eine Teilansicht der Vorrichtung aus Fig. 1 in schematischer Prinzipdarstellung. Dargestellt sind hier zusätzlich die schematischen Strahlengänge für die Beleuchtung und die Bildgebung. Die Beleuchtungseinheit ist mit Lichtquellen 3 so aufgebaut, dass die Lichtquellen um das Objektiv 2 angeordnet sind. Je nach Öffnungswinkel (Objektiv mit Festbrennweite) oder Öffnungswinkelbereich (Varioobjektiv), ist die Beleuchtungseinheit mit Lichtquellen optisch strahlformenden Elementen versehen, so dass das Untersuchungsgebiet möglichst homogen ausgeleuchtet wird (Ausleuchtungsgebiet 14). Das Objekt oder der Körper 1 wird im Abbildungsbereich 13 über das Eingangsobjektiv 2 mit der Bildebene auf den Farbkamerasensor 6 und die Blende 7 des Spektrometers 11 abgebildet, dass das Bild des Untersuchungsgebietes 13 den gesamten Farbkamerasensor 6 und den Kamerasensor 10 (Fig. 1) bedeckt.

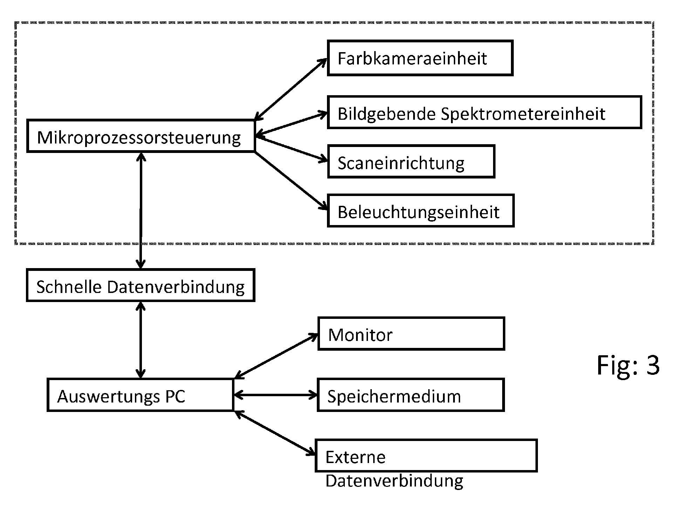

Fig. 3 zeigt ein Blockschaltbild einer bevorzugten Ausführungsform der Vorrichtung. In der Vorrichtung übernimmt eine Datenverarbeitungseinrichtung die gesamte Steuerung, Datensynchronisation und Datenauslese und -zwischenspeicherung. Die Datenverarbeitungseinrichtung übernimmt zum einen die Parametrierung des Farbkamerasensors 6, die digitale Datenaufnahme des Farbkamerasensors 6, die Parametrierung des monochromen Kamerasensors (6) der hyperspektralen Aufnahmevorrichtung (unter Parametrierung wird hier das Einstellen der Register des Kamerasensors, von Gain, Skipping, Binning, Belichtungszeit, ROI, Pixelrate etc. verstanden), die Datenaufnahme des Kamerasensors 6, die synchronisierte Steuerung der mechanischen Antriebseinrichtung und die Steuerung der Lichtquellen 3. Weiterhin kann die Datenverarbeitungseinrichtung optional auch eine Datenreduzierung, Datenzwischenspeicherung oder Datenvorverarbeitung durchführen.

Die von dem Kamerasensor 10 und dem zweiten Kamerasensor 6 aufgenommen Daten werden von der Datenverarbeitungseinrichtung gesammelt und gespeichert. Ferner ist die Datenverarbeitungseinrichtung dazu eingerichtet, Antriebseinrichtungen anzusteuern, um das Spektrometer 11 nach jeder Aufnahme eines durch die Blende 7 selektierten spaltförmigen Bereichs des Bildes des Untersuchungsgebietes um eine Strecke zu verschieben, die der Spaltbreite der Blende entspricht. Dazu kann das Spektrometer an einer Linearführung aufgehängt oder auf Schienen gelagert sein. Die Antriebseinrichtungen können durch Stellmotoren oder anderweitige steuerbare Aktuatoren realisiert sein. Die Datenverarbeitungseinrichtung ist dazu eingerichtet, diese Antriebseinrichtung nach jeder Aufnahme eines spaltförmigen Bereichs des Bildes des Untersuchungsgebietes zu betätigen, um das Spektrometer um eine Wegstrecke in der von der Längsrichtung der Blende verschiedenen zweiten Richtung zu verschieben, die z.B. der Spaltbreite der Blende 7 entsprechen kann (die Schrittweite kann auch kleiner als die Spaltbreite sein und der Pixelgröße entsprechen). Gleichzeitig speichert die Datenverarbeitungseinrichtung zu jeder Aufnahme eines spaltförmigen Bereichs des Bildes des Untersuchungsgebietes die relative Position in der Richtung y, in der das Spektrometer in der Bildebene verfahren ist, so dass jedem Spektrum ein Paar von Ortskoordinaten x, y des Bildes des Untersuchungsgebietes zugeordnet ist.

In der Datenverarbeitungseinrichtung wird eine Datenvorverarbeitung der Hyperspektralbilder vorgenommen und es erfolgt eine chemometrie-basierte Auswertung der Hyperspektralbilder. Durch den sehr hohen Datenstrom kann es sinnvoll sein, in der Datenverarbeitungseinrichtung neben üblichen Einheiten wie Mikroprozessoren und einem PC zusätzliche, gegebenenfalls in die PC-Einheit integrierte spezielle Prozessoren (z.B. Graphikprozessoren) vorzusehen, in die bestimmte Prozesse wie Graphikerzeugung, Koordinatentransformationen, Bildenzerrungen, und chemometrische Algorithmen und dergleichen ausgelagert werden. Weiterhin kann eine Aufarbeitung und Optimierung der Farbbilder erfolgen. Diese Bilddatensätze können anschließend auf einem Monitor angezeigt, auf einem Speichermedium abgelegt oder über eine weitere externe Datenverbindung an andere Systeme weitergegeben werden. Die Datenverarbeitungseinrichtung muss nicht in einer Einheit zusammengefasst sein, sondern kann auch durch verteilt angeordnete Prozessoren und Computer realisiert sein.

Die Darstellung der Aufnahmen kann zum Beispiel so erfolgen, dass neben der farbigen Darstellung des Untersuchungsgebiets aufgenommen durch den optischen Farbkamerasensor ein oder mehrere Analysedarstellungen angezeigt werden, die aus dem Hyperspektralbild gewonnen worden ist. Solche Analysedarstellungen können z.B. zweidimensionale Darstellungen des Untersuchungsgebietes sein, in denen die Konzentrationen bestimmter chemischer Substanzen kodiert ortsaufgelöst dargestellt sind, z.B. die Konzentration eines bestimmten chemischen Stoffes kodiert in einer Farbskala von 0 bis 100% über das Untersuchungsgebiet.

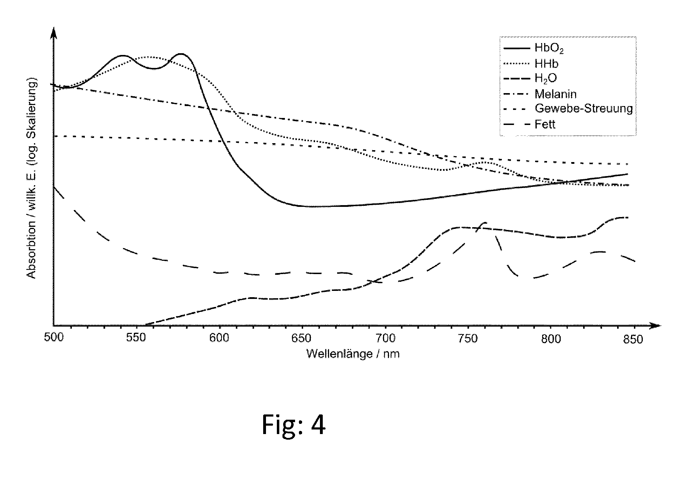

Fig. 4 zeigt die Einzelspektren von reinem, oxygeniertem Hämoglobin, von deoxygeniertem Hämoglobin, Wasser, dem Hautpigment Melanin, Fett und von typischen Streueigenschaften menschlichen Gewebes. Die Auftragung der Absorption in der Y-Achse ist logarithmisch vorgenommen. Die Darstellung soll hauptsächlich die typischen Verläufe der Absorptionskurven und der charakteristischen Absorptionsbanden zeigen. Da die Konzentrationen in Hyperspektralbildern der einzelnen chemischen Komponenten um Größenordnungen variieren können und unbekannte Substanzen zu den Spektren beitragen, ist in der Praxis eine kombinierte chemometrische Auswertung anhand der Adsorptionsspektren und anhand der zweiten Ableitungen der Spektren d²(I(λ)/Iᵢₙ(λ))/dλ² in der Praxis sinnvoll. An die gemessenen Spektren können vorab bestimmte Einzelspektren reiner Substanzen angepasst werden, wobei die Anpassung an die Spektren selbst, in deren zweiten Ableitungen oder kombiniert sowohl an Spektren und in deren zweiten Ableitungen erfolgen kann. Dazu wird ein multivariates statistisches Analyseverfahren verwendet, um die Beiträge der Einzelsubstanzen zu den erfassten Spektren zu ermitteln und daraus insbesondere den relativen Beitrag von oxygeniertem Hämoglobin zu deoxygeniertem Hämoglobin (Oxygenierung), die Gesamthämoglobinkonzentration und Konzentrationen der weiteren Substanzen zu bestimmen. Anschaulich wird in einem solchen Analyseverfahren das gemessene Spektrum als Linearkombination der Einzelspektren der reinen Substanzen dargestellt und die Koeffizienten der Einzelspektren in derjenigen Linearkombination der Einzelspektren ermittelt, die die beste Übereinstimmung mit dem gemessenen Spektrum liefert. Es sollten daher möglichst die Einzelspektren aller Substanzen vorab gemessen und gespeichert werden, die in dem untersuchten Wellenlängenbereich relevant sein können. Soweit die zweiten Ableitungen der Spektren betrachtet werden, müssen natürlich nur solche Substanzen berücksichtigt werden, deren Spektren in zweiter Ableitung in dem fraglichen Wellenlängenbereich von 0 verschieden sind. Z.B. brauchen Melanin und die Gewebestreuung in dem hier vorzugsweise betrachteten Spektralbereich von 500 nm bis 850 nm nicht berücksichtigt zu werden, da sie keine signifikanten Steigungsänderungen in den Spektren aufweisen und in der zweiten Ableitung der Spektren daher näherungsweise 0 sind. Bei der Aufnahme der Einzelspektren ist insbesondere beim Wasserspektrum auf dessen Temperaturabhängigkeit zu achten, d.h. die Einzelspektren sollten bei einer Körpertemperatur entsprechenden Temperatur aufgenommen werden.

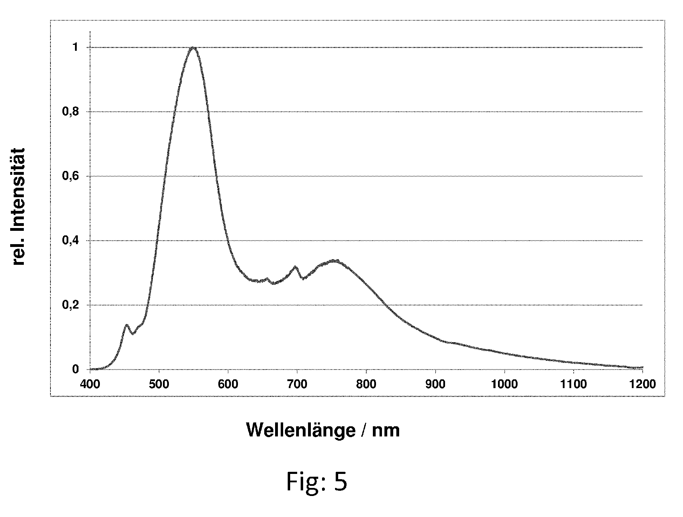

Fig. 5 zeigt ein beispielhaftes Emissionsspektrum einer Breitband-LED. Dieses Emissionsspektrum wurde nach folgenden Kriterien optimiert. Das Spektrum muss die Spektralbereiche für die Farbbildgebung enthalten (460 nm Blau, 530 nm Grün und 620 nm Rot). Weiterhin wurde das Emissionsspektrum entgegengesetzt zur typischen Absorption der Haut gestaltet. So wird viel Strahlung im Spektralbereich von 500 bis 600 nm emittiert, wo die Absorptionen durch das Melanin der Haut und das Hämoglobin sehr hoch sind. So findet eine optimierte Ausleuchtung des Untersuchungsgebietes eines menschlichen Körperteils zur Aufnahme eines Hyperspektralbildes statt.

## Patentansprüche

1. Vorrichtung zur Aufnahme eines Hyperspektralbildes eines Untersuchungsgebietes eines Körpers, mit
einer Lichtquelle (3) zur Bestrahlung des Untersuchungsgebietes mit Licht mit für die gewünschte spektroskopische Analyse geeigneter spektraler Verteilung,
einem Eingangsobjektiv (2) zur Erzeugung eines Bildes des Untersuchungsgebietes in einer Bildebene,
einem Spektrometer (11), das eine in der Bildebene angeordnete schlitzförmige Blende (7) zur Selektion eines schlitzförmigen Bereichs des Bildes des Untersuchungsgebietes, ein dispersive Element (9), das so aufgebaut und angeordnet ist, dass die dispersive Auffächerung des durch die Blende hindurch tretenden Lichts in einer von der Längsrichtung der Blende verschiedenen Richtung erfolgt, und einen Kamerasensor (10) aufweist, der so aufgebaut und angeordnet ist, dass er ein Beugungsbild des dispersive Elements mit der Längsrichtung der Blende in einer ersten Richtung und der dispersiven spektralen Auffächerung des Lichtes in der von der ersten verschiedenen Richtung auf seiner Kamerasensorfläche aufnimmt, und
einer mit dem Kamerasensor verbundenen Datenverarbeitungseinrichtung, die dazu eingerichtet ist, die von dem Kamerasensor (10) empfangenen Signale als eine Vielzahl von Spektren mit jeweils zugeordneter Ortskoordinate x entlang der Längsrichtung X der Blende zu speichern,
wobei die Vorrichtung dazu eingerichtet ist, in einer von der Längsrichtung X verschiedenen zweiten Richtung Y in der Bildebene aufeinanderfolgende schlitzförmige Bereiche des Bildes des Untersuchungsgebietes mit zugeordneter Ortskoordinate y in der zweiten Richtung aufzunehmem, und die Datenverarbeitungseinrichtung dazu eingerichtet ist, die Spektren jeweils zu speichern und die aufgezeichneten Spektren mit den zugeordneten Ortskoordinaten x, y zu einem Hyperspektralbild des Untersuchungsgebietes zusammenzufassen,
**dadurch gekennzeichnet, dass** das Spektrometer (11) in der zweiten Richtung Y verfahrbar relativ zum Eingangsobjektiv (2) gelagert ist, eine Antriebseinrichtung zum gesteuerten Verfahren und Einstellen der Position des Spektrometers (11) in der zweiten Richtung Y vorhanden ist und dass die Datenverarbeitungseinrichtung dazu eingerichtet ist, das Spektrometer (11) mit der Blende (7) in der Bildebene des Untersuchungsgebietes entlang der Bildebene schrittweise sukzessive zu verfahren, um so sukzessive aufeinanderfolgende schlitzförmige Bereiche des Bildes des Untersuchungsgebietes mit Spektren der Bildelemente mit jeweils zugeordneten Ortskoordinaten x, y aufzunehmen und zu dem Hyperspektralbild zusammenzusetzen.

2. Vorrichtung nach Anspruch 1, **dadurch gekennzeichnet, dass** im Strahlengang zwischen dem Eingangsobjektiv (2) und dem Spektrometer (11) ein Strahlteiler angeordnet ist, der die Strahlen aus dem Untersuchungsgebiet hinter dem Eingangsobjektiv (2) aufteilt und die abgeteilten Teilstrahlen ablenkt und auf einen zweiten Kamerasensor (6) richtet, der in einer zweiten Bildebene angeordnet ist, um parallel zu dem Hyperspektralbild des Untersuchungsgebietes ein optisches Bild des Untersuchungsgebietes aufzunehmen und auf einer Anzeigeeinrichtung zur Anzeige zu bringen.

3. Vorrichtung zur Aufnahme eines Hyperspektralbildes nach Anspruch 1 oder 2, **dadurch gekennzeichnet, dass** die Datenverarbeitungseinrichtung dazu eingerichtet ist, die Spektren des Hyperspektralbildes auf vorgegebene Kriterien hin zu untersuchen und Spektren von Bildelementen nach Maßgabe der Ergebnisse der Untersuchungen Bewertungsgrößen zuzuordnen und ein zweidimensionales Analysebild der Bildelemente des Untersuchungsgebietes zu erzeugen, in dem den Bildelementen in Abhängigkeit von den zugehörigen Bewertungsgröße optische Kodierungen zugeordnet ist, und so ein optisch kodiertes Analysebild in einer zweidimensionalen Form zu erzeugen und auf der Anzeigeeinrichtung zur Anzeige zu bringen.

4. Vorrichtung zur Aufnahme eines Hyperspektralbildes nach einem der Ansprüche 2 bis 3, **dadurch gekennzeichnet, dass** die Datenverarbeitungseinrichtung weiter dazu eingerichtet ist, neben einer oder mehreren optisch kodierten Formen der Analysebilder auf der Anzeigeeinrichtung eine Darstellung des von dem zweiten Kamerasensor (6) aufgenommenen optischen Bildes des Untersuchungsgebietes zur Anzeige zu bringen.

5. Vorrichtung zur Aufnahme eines Hyperspektralbildes nach einem der vorhergehenden Ansprüche, **dadurch gekennzeichnet, dass** das dispersive Element des Spektrometers ein Gitter ist (9).

6. Vorrichtung zur Aufnahme eines Hyperspektralbildes nach Anspruch 5, **dadurch gekennzeichnet, dass** das Gitter (9) des Spektrometers ein Blaze-Gitter ist, das dazu optimiert ist, das dispergierte Licht im Wellenlängenbereich von 500 bis 1.000 nm in einen vorgegebenen Winkelbereich konzentriert abzugeben, und dass das Blaze-Gitter ein Reflexions- oder Transmissionsgitter mit asymmetrisch sägezahnförmig gestalteter Oberfläche oder ein holographisches Gitter ist.

7. Vorrichtung nach Anspruch 6, **dadurch gekennzeichnet, dass** das Blaze-Gitter ein VPH-Gitter (Volume Phase Holographic Grating) ist.

8. Vorrichtung nach einem der vorhergehenden Ansprüche, **dadurch gekennzeichnet, dass** die Datenverarbeitungseinrichtung Zugriff auf vorab gemessene und gespeicherte Spektren von reinen, im Körper vorhandenen chemischen Substanzen hat und weiter dazu eingerichtet ist, nach einem Verfahren der multivariaten Statistik die gemessenen Spektren eines Hyperspektralbildes aus den Anteilen der gespeicherten Spektren der chemischen Substanzen zusammenzusetzen und daraus Informationen über chemische Eigenschaften regional zu bestimmen.

9. Vorrichtung nach einem der vorhergehenden Ansprüche, **dadurch gekennzeichnet, dass** die Datenverarbeitungseinrichtung dazu eingerichtet ist, die zweiten Ableitungen der aufgenommenen Spektren nach der Wellenlänge zu bilden und diesen zweiten Ableitungen auf vorgegebene Merkmale hin zu untersuchen.

10. Vorrichtung nach einem der Ansprüche 2 bis 9, **dadurch gekennzeichnet, dass** die Datenverarbeitungseinrichtung dazu eingerichtet ist, während der Aufnahme eines Hyperspektralbildes sukzessive mehrere optische Aufnahmen des Untersuchungsgebietes durch den zweiten Kamerasensor aufzunehmen, zu speichern und daraufhin zu untersuchen, ob während der Abtastung des Untersuchungsgebiets in den optischen Bildaufnahmen des Untersuchungsgebiets Bildverschiebungen feststellbar sind, die über eine Schwellenverschiebung hinausgehen, und bei Feststellung einer solchen Bildverschiebung die Aufnahme des Hyperspektralbilds abzubrechen oder eine Warnung auszugeben, dass bei der Aufnahme des Hyperspektralbilds eine Verschiebung des untersuchten Körperteils oder Objekts um mehr als eine Schwellenverschiebung stattgefunden hat.

11. Verfahren zur Aufnahme eines Hyperspektralbildes eines Untersuchungsgebietes eines Körpers, bei dem
das Untersuchungsgebiet mit einer Lichtquelle (3) mit Licht mit für die gewünschte spektroskopische Analyse geeigneter spektraler Verteilung bestrahlt wird,
mit einem Eingangsobjektiv (2) ein Bild des Untersuchungsgebiets in einer Bildebene erzeugt wird,
mit einem Spektrometer (11), das eine in der Bildebene angeordnete schlitzförmige Blende (7) zur Selektion eines schlitzförmigen Bereichs des Bildes des Untersuchungsgebietes, ein dispersives Element (9), vorzugsweise ein Gitter, das so aufgebaut und angeordnet ist, dass die dispersive Auffächerung des durch die Blende hindurch tretenden Lichts durch das Gitter in einer von der Längsrichtung der Blende verschiedenen Richtung erfolgt, und einen Kamerasensor (10) aufweist, der so aufgebaut und angeordnet ist, dass er ein Beugungsbild des dispersiven Elements mit der Längsrichtung der Blende in einer ersten Richtung und der dispersiven spektralen Auffächerung des Lichtes in einer von der ersten verschiedenen Richtung auf seiner Kamerasensorfläche aufnimmt, eine Vielzahl von Spektren von Bildelementen entlang der Längsrichtung der Blende mit jeweils zugeordneter Ortskoordinate x entlang der Längsrichtung X der Blende aufgenommen wird,
die aufgenommenen Spektren in einer mit dem Spektrometer (11) verbundenen Datenverarbeitungseinrichtung mit jeweils zugeordneter Ortskoordinate x entlang der Längsrichtung X der Blende gespeichert werden,
in einer von der Längsrichtung X verschiedenen zweiten Richtung Y in der Bildebene aufeinanderfolgende schlitzförmige Bereiche des Bildes des Untersuchungsgebietes mit zugeordneter Ortskoordinate y in der zweiten Richtung Y mit dem Spektrometer untersucht und die zugehörigen Spektren mit jeweils zugeordneten Ortkoordinaten x, y in der Datenverarbeitungseinrichtung gespeichert werden,
die aufgezeichneten Spektren mit den zugeordneten Ortskoordinaten x, y zu einem Hyperspektralbild des Untersuchungsgebietes zusammengefasst werden,
**dadurch gekennzeichnet,**
**dass** das Spektrometer (11) in der zweiten Richtung Y verfahrbar relativ zum Eingangsobjektiv (2) gelagert ist und dass das Spektrometer angetrieben von einer Antriebseinrichtung zum gesteuerten Verfahren und Einstellen der Position des Spektrometers (11) in der zweiten Richtung Y schrittweise die Bildebene des Bildes des Untersuchungsgebietes abtastet, um so sukzessive aufeinanderfolgende schlitzförmige Bereiche des Bildes des Untersuchungsgebietes mit Spektren der Bildelemente mit jeweils zugeordneten Ortskoordinaten x, y aufzunehmen und zu dem Hyperspektralbild zusammenzusetzen.
